# EUROPEAN PATENT SPECIFICATION

(11) **EP 0 599 492 B1**
(45) Date of publication and mention of the grant of the patent: **05.06.1996**
(21) Application number: 93308668.8
(22) Date of filing: 29.10.1993
(51) Int. Cl.: C09C 1/36, A61K 7/42

(54) **Coated titanium dioxide**
Beschichtetes Titandioxid
Dioxyde de titane revêtu

(30) Priority: 24.11.1992 GB 9224529
(43) Date of publication of application: 01.06.1994
(73) Proprietor: TIOXIDE GROUP SERVICES LIMITED, London W14 0QL (GB)
(72) Inventor: Sayer, Andrew Timothy, Stockton on Tees, Cleveland TS16 9HL (GB)
(74) Representative: Jackson, John Derek

(56) References cited:
- EP-A- 0 535 972
- GB-A- 2 205 088
- DATABASE WPI Week 9309, Derwent Publications Ltd., London, GB; AN 93-071009

## Description

This invention relates to particulate titanium dioxide and in particular to titanium dioxide having a small particle size and a coating of zinc oxide.

The use of titanium dioxide with a particle size within the range 0.005 to 0.15 micrometre to provide protection from ultraviolet light is known. It is also known that a mixture of this titanium dioxide with zinc oxide having a similar particle size can be used to provide protection over a wider range of wavelengths than is possible with titanium dioxide alone. Balanced protection over a wide range of wavelengths is useful, for example, in sunscreens since it is now considered desirable to protect skin from UVB radiation (wavelength 290-320 nm) and UVA radiation (wavelength 320-400 nm).

An object of this invention is to provide an effective and convenient means of providing such balanced protection.

According to the invention a particulate material comprises titanium dioxide having an average particle size within the range 0.005 to 0.15 micrometre and having a coating comprising zinc oxide or hydrous zinc oxide in an amount of at least 100 per cent by weight with respect to the weight of titanium dioxide said material being substantially transparent to visible light and substantially absorbent to ultraviolet light.

Included within the scope of the invention is particulate material in which the amount of zinc oxide or hydrous oxide present is considerably greater than the amount of titanium dioxide with the titanium dioxide forming a relatively small core within the particle. For the purposes of this invention such particles are considered to be titanium dioxide coated with zinc oxide or hydrous oxide.

The particles of titanium dioxide may be of the rutile, anatase or amorphous form and each particle may be composed of aggregated smaller particles or crystals or, ideally, each particle is a single particle of the size chosen to be advantageous.

The average particle size of the titanium dioxide is from 0.005 to 0.15 micrometre and this size refers to the dimension of the uncoated particles. Where the particles are substantially spherical then this size will be taken to represent the diameter. However, since the invention also encompasses the use of non-spherical particles then in such cases the size refers to the largest dimension. One preferred product is acicular in shape and has a ratio of largest dimension to shortest dimension of from 8:1 to 2:1.

Preferably the particles of titanium dioxide have an average size within the range 0.01 to 0.06 micrometre and most preferably within the range 0.01 to 0.03 micrometre when they are substantially spherical in shape. For particles having an acicular shape then the average largest dimension preferably is within the range 0.02 to 0.15 micrometre and most preferably within the range 0.02 to 0.10 micrometre.

Also it is preferred that the particles of titanium dioxide have a narrow size distribution and the most preferred particles have from 80 per cent to 100 per cent of the particles with a size of from 0.005 to 0.15 micrometre.

The particulate material of the invention has a coating of zinc oxide or hydrous zinc oxide on the titanium dioxide, the amount of zinc oxide or hydrous oxide being at least 100 per cent of the amount of titanium dioxide. Preferably the amount of zinc oxide or hydrous oxide present is at least 300 per cent by weight. The purpose for which the coated titanium dioxide is designed to be employed determines to some extent the preferred amount of zinc oxide or hydrous oxide present. For example, a powder suitable for use in a plastics composition preferably has a coating of zinc oxide or hydrous oxide comprising 300 to 1000 per cent by weight of the titanium dioxide. A product suitable for use in cosmetics preferably comprises 750 to 2000 weight per cent zinc oxide or hydrous oxide with respect to titanium dioxide and most preferably the amount of zinc oxide or hydrous oxide is from 1200 to 1800 per cent by weight of titanium dioxide.

The particulate titanium dioxide of the present invention may be formed by any suitable process. Typical processes may involve hydrolysis of an appropriate titanium compound such as a titanium sulphate, a titanium chloride or an organic or inorganic titanate or oxidation of an oxidisable titanium compound, for example in the vapour state.

A typical process involves the preparation of a solution of a soluble titanium salt which is then hydrolysed to form hydrous titanium oxide. The solution can be that obtained in the so-called "sulphate" process for the manufacture of titanium dioxide pigment in which a titaniferous ore is digested with concentrated sulphuric acid and the digestion cake is dissolved in water or dilute acid to produce a solution of titanyl sulphate. During the process additional process stages of clarification and reduction are usually employed. Hydrolysis of the titanyl sulphate solution produces a precipitate of hydrous titania which is sometimes called "pulp". Soluble iron compounds remain in solution and the precipitated pulp is treated by neutralisation and washing to an appropriate degree of impurity level.

A suitable form of acicular titanium dioxide is produced by treating the precipitated pulp with, say, sodium hydroxide and subsequently hydrochloric acid.

The titanium dioxide may be dried before a coating of zinc oxide is produced on the particles but it is convenient to coat the precipitated and washed pulp.

Usually prior to coating it is preferred to mill the titanium dioxide to an appropriate particle size falling within the range specified hereinbefore. Milling conveniently can be effected in a wet milling process employing a grinding medium such as sand which can be separated easily and effectively from the milled pulp. Milling, preferably, is carried out in the presence of a dispersing agent. Usually, this dispersing agent is an inorganic dispersing agent such as an alkali metal silicate, for example sodium silicate, although an organic dispersing agent such as monoisopropanolamine or a polyacrylate is also useful.

The titanium dioxide is then treated to deposit zinc oxide or hydrous oxide in the specified amount. Typically, an aqueous dispersion of titanium dioxide containing a hydrolysable salt of zinc is treated with a reagent which effects hydrolysis of the zinc salt to form zinc oxide or hydrous oxide. Useful salts of zinc include zinc chloride, zinc sulphate and zinc nitrate. An aqueous solution of these salts is acidic and zinc oxide or hydrous oxide is precipitated from such a solution by the addition of an alkali.

In a typical process for forming a coating of zinc oxide on the titanium dioxide a dispersion of the titanium dioxide at a concentration of 80 to 250 grams per litre is formed. The dispersion is heated with stirring to between 40°C and 70°C and this temperature and vigorous stirring is maintained throughout the coating process. A solution of zinc chloride containing sufficient zinc chloride to form the desired amount of zinc oxide is slowly added to the dispersion. After all the zinc chloride has been added the pH is raised slowly by the addition of alkali to a value between 9 and 10. Precipitation of zinc oxide commences at a pH value of approximately 4.5.

In an alternative process the titanium dioxide dispersion is adjusted to a pH between 9 and 10 and zinc chloride is slowly added, the pH being maintained between 9 and 10 during this addition by the simultaneous addition of an alkali such as sodium hydroxide. After addition is complete the pH of the dispersion is raised, if necessary, to between 9.5 and 10.

Generally, the coated product is washed to remove soluble salts by, for example, reslurrying, with water at 60°C to 70°C, allowing the solid to settle and decanting off the supernatant liquor. This washing is repeated as often as necessary. After separation by, for example, filtration the product is dried typically by heating overnight at 120°C.

The product may also be further treated by heating at a temperature of from 200°C to 650°C, preferably 200°C to 350°C.

The products of the present invention have the property of absorbing and reflecting ultraviolet light and transmitting visible light. The absorption and reflection of ultraviolet light extends over a large portion of the ultraviolet spectrum known as UVA and UVB radiation. The absorbancy plus reflectance, collectively termed attenuation, of the particles can be expressed as an extinction coefficient and when so expressed is substantially independent of a medium in which the particles are dispersed. The attenuation of UVA light can be estimated by attenuation at, say, 360 nm wavelength and attenuation of UVB light can be estimated by attenuation at, say, 308 nm wavelength. Generally speaking, when adequately dispersed the products of the current invention have a minimum extinction coefficient at 308 nm (E(308)) of at least 15 litres per gram per centimetre and a minimum extinction coefficient at 360 nm (E(360)) of at least 10 litres per gram per centimetre. Preferably E(308) is at least 20 litres per gram per centimetre and E(360) is at least 13 litres per gram per centimetre. The extinction coefficient at 524 nm (E(524)) can be used as an indication of the transparency of the product to visible light. Preferably E(524) is less than 5 litres per gram per centimetre and most preferably E(524) is less than 3 litres per gram per centimetre.

The products of the invention can be sold or used in the dry state or converted into a dispersion in water or an organic medium before use. For example, a very useful product is obtained by milling the product of this invention in an oil in the presence of a dispersing agent. Such a dispersion can provide a convenient basis for the formulation of ultraviolet light attenuating compositions such as sunscreens.

The products of the invention find use in a wide variety of applications wherein it is important to maintain transparency to visible light while substantially preventing transmission of ultraviolet light to a surface. Cosmetics, sun creams, plastics films, wood coatings and other coating compositions are a small number of such applications.

The balanced protection provided against UVA and UVB light is particularly useful in sunscreen products. Usually, the coated products of the invention have better optical properties than a mixture of titanium dioxide and zinc oxide in similar proportions. The particulate titanium dioxide which forms the core of the particles of the invention substantially retains the optical characteristics of uncoated titanium dioxide even when the zinc oxide or hydrous oxide coating amounts to a large proportion of the particulate material.

The invention is illustrated by the following examples.

### EXAMPLE 1

353 grams of titania pulp (120 grams TiO₂) obtained by digestion of ilmenite with sulphuric acid, hydrolysis of the titanyl sulphate solution produced, treatment of the precipitate formed with sodium hydroxide to obtain sodium titanate and reaction of the sodium titanate with hydrochloric acid was sand milled at 100 grams per litre for 2 hours with an amount of sodium silicate equivalent to 5% SiO₂ by weight on TiO₂, at pH 10. The grinding medium was Ottawa sand (size distribution 0.7 mm to 0.9 mm) which was removed and washed at the end of the milling period. The washings were added to the milled titania dispersion.

The titania dispersion (95 grams per litre) was heated to 55°C and vigorously stirred.

To the stirred dispersion aqueous zinc chloride solution (equivalent to 1000 grams per litre zinc chloride) was added dropwise in an amount sufficient to introduce the equivalent of 300% by weight zinc oxide on weight of TiO₂ over a period of 60 minutes while the temperature of the dispersion was maintained between 50°C and 60°C. Approximately 0.6 litres of the solution was added.

Simultaneously sodium hydroxide (400 grams per litre) was co-additioned such that the dispersion was maintained at pH 9.7.

After the addition was completed the dispersion was allowed to age for 30 minutes at 50°C - 60°C whilst stirring was maintained.

The dispersion was allowed to cool and settle. The soluble zinc ion concentration in the supernatant liquor was measured at 1.2 grams per litre.

The supernatant liquor was removed and the coated product was washed with de-ionised water at 60°C until the ionic conductivity of the dispersion was less than 300 microsiemens per centimetre.

The coated product was filtered and dried at 120°C for 6 hours.

Electron microscopy showed that the product was acicular rutile titanium dioxide having an average size of 0.02 x 0.08 micrometre. The coating comprised 5% by weight SiO₂ and 300% by weight ZnO based on TiO₂ and the average particle size of the coated particles was 0.03 x 0.10 micrometre.

The product was tested in aqueous media by adding 2.5 grams of product to 25 ml of de-ionised water containing 10% by weight sodium polyacrylate dispersant based on product.

The product was then dispersed in a small sand mill for 45 minutes with fine zirconia, having a classified particle size of from 212 micrometre to 180 micrometre, as the grinding medium.

The dispersion was removed from the mill and the milled, coated titanium dioxide separated from the grinding medium and diluted with de-ionised water to 0.04 grams per litre.

The diluted dispersion was then exposed in a spectrometer (Perkin Elmer Lambda 2) with a 1 cm path length and the attenuation of UV and visible light measured.

Extinction coefficients at three wavelengths were calculated from A = E.c.l, where A = attenuation, E = extinction coefficient in litres per gram per cm, c = concentration in grams per litre, and l = path length in cm.

The results were

| E(524 nm) | E(360 nm) | E(308 nm) |
|---|---|---|
| 1.3 | 10.2 | 20.2 |

Extinction coefficients of a dispersion of a mixture of 1 part by weight titanium dioxide having a similar particle size to that used in Example 1 and 3 parts by weight zinc oxide having an average particle size of 0.06 micrometre were measured at the same wavelengths. The following values were obtained

| E(524 nm) | E(360 nm) | E(308 nm) |
|---|---|---|
| 2.2 | 12.3 | 18.5 |

The extinction coefficient for visible light was higher than for the coated powder of Example 1 although the extinction coefficients for ultraviolet light were similar.

### EXAMPLE 2

Example 1 was repeated with the following differences. The amount of zinc chloride used was adjusted to produce a titanium dioxide coated with 5% by weight SiO₂ and 1500% by weight ZnO on TiO₂ and the zinc chloride was all added to the titanium dioxide dispersion before any sodium hydroxide was added. Sufficient sodium hydroxide was added subsequent to the zinc chloride addition to ensure that the final pH of the dispersion was 9.7.

Electron microscopy showed that the uncoated product was acicular rutile titanium dioxide having an average size of 0.02 x 0.08 micrometre. The coating comprised 5% by weight SiO₂ and 1500% by weight ZnO based on TiO₂ and the average particle size of the coated particles was 0.07 x 0.13 micrometre.

The extinction coefficients obtained were

| E(524 nm) | E(360 nm) | E(308 nm) |
|---|---|---|
| 0.8 | 12.9 | 17.1 |

For comparison, a blend of 1 part by weight titanium dioxide and 15 parts by weight zinc oxide had the following extinction coefficients

| E(524 nm) | E(360 nm) | E(308 nm) |
|---|---|---|
| 2.4 | 12.4 | 12.7 |

## Claims

1. Particulate material comprising titanium dioxide having a coating comprising zinc oxide or hydrous zinc oxide characterised in that the particles of titanium dioxide have an average particle size within the range 0.005 to 0.15 micrometre and the zinc oxide or hydrous zinc oxide coating is present in an amount of at least 100 per cent by weight with respect to the weight of titanium dioxide, said material being substantially transparent to visible light and substantially absorbent to ultraviolet light.

2. Particulate material according to claim 1 characterised in that the titanium dioxide particles are acicular in shape and have a ratio of largest dimension to shortest dimension of from 8:1 to 2:1.

3. Particulate material according to claim 1 or 2 characterised in that the particles have an acicular shape and the average largest dimension is within the range 0.02 to 0.15 micrometre.

4. Particulate material according to claim 1 characterised in that the titanium dioxide particles are substantially spherical in shape and have an average diameter within the range 0.01 to 0.06 micrometre.

5. Particulate material according to claim 1 characterised in that from 80 per cent to 100 per cent of the particles have a size of from 0.005 to 0.15 micrometre.

6. Particulate material according to any one of the preceding claims characterised in that the amount of zinc oxide or hydrous zinc oxide is from 300 per cent to 1000 per cent by weight with respect to weight of titanium dioxide.

7. Particulate material according to any one of claims 1 to 5 characterised in that the amount of zinc oxide or hydrous zinc oxide is from 750 per cent to 2000 per cent by weight with respect to weight of titanium dioxide.

8. Particulate material according to any one of the preceding claims characterised in that, when dispersed, the material has an extinction coefficient for light with a wavelength of 308 nanometres (E(308)) of at least 15 litres per gram per centimetre and an extinction coefficient for light with a wavelength of 360 nanometres (E(360)) of at least 10 litres per gram per centimetre.

9. Particulate material according to any one of the preceding claims characterised in that, when dispersed, the material has an extinction coefficient for light with a wavelength of 524 nanometres (E(524)) of less than 5 litres per gram per centimetre.

10. Particulate material according to any one of the preceding claims characterised in that the material is in the form of a dispersion in oil and the dispersion is prepared by milling the particulate material in the oil in the presence of a dispersing agent.

11. A process of preparing particulate material comprising forming an aqueous dispersion of titanium dioxide particles in the presence of a hydrolysable salt of zinc and effecting hydrolysis of the zinc salt so as to form on the particles of titanium dioxide a coating of zinc oxide or hydrous zinc oxide characterised in that the titanium dioxide has an average particle size within the range 0.005 to 0.15 micrometre, the zinc salt is employed in an amount sufficient to deposit on the titanium dioxide particles a coating of zinc oxide or hydrous zinc oxide comprising at least 100 per cent by weight with respect to the weight of titanium dioxide and the coated titanium dioxide so formed is substantially transparent to visible light and substantially absorbent to ultraviolet light.

12. A process according to claim 11 characterised in that the aqueous dispersion of titanium dioxide particles has a concentration of 80 to 250 grams per litre.

13. A process according to claim 11 or 12 characterised in that sufficient zinc chloride to form a coating of zinc oxide on the titanium dioxide particles in a desired amount is added to the dispersion of titanium dioxide particles and the zinc oxide is precipitated by subsequent addition of an alkali until the pH value of the dispersion is between 9 and 10.

14. A process according to claim 11 or 12 characterised in that the dispersion of titanium dioxide particles is formed at a pH value between 9 and 10, zinc chloride solution is slowly added to this dispersion and the pH of the dispersion is maintained at a value between 9 and 10 by the simultaneous addition of an alkali.

15. A process according to any one of claims 11 to 14 characterised in that the dispersion of titanium dioxide particles is subjected to a milling process before formation of the coating of zinc oxide or hydrous oxide is commenced.

16. A process according to claim 15 characterised in that the milling is carried out in the presence of a dispersing agent comprising an alkali metal silicate, an alkanolamine or a polyacrylate.

17. A process according to any one of claims 11 to 16 characterised in that the coated titanium dioxide is washed by reslurrying with water at 60°C to 70°C.

18. A process according to any one of claims 11 to 17 characterised in that the coated titanium dioxide is further treated by heating at a temperature of from 200°C to 650°C.

## Patentansprüche

1. Teilchenförmiges Material, umfassend Titandioxid mit einer Beschichtung, welche Zinkoxid oder wasserhaltiges Zinkoxid umfaßt,
dadurch gekennzeichnet,
daß die Teilchen des Titandioxids eine mittlere Teilchengröße im Bereich von 0,005 bis 0,15 Mikrometer aufweisen und die Beschichtung aus Zinkoxid oder wasserhaltigem Zinkoxid in einer Menge von mindestens 100 Gew.-% bezüglich des Gewichts von Titandioxid vorliegt, wobei das Material im wesentlichen für sichtbares Licht transparent ist und im wesentlichen Ultraviolettlicht absorbiert.

2. Teilchenförmiges Material gemäß Anspruch 1,
dadurch gekennzeichnet,
daß die Titandioxidteilchen nadelförmig sind und ein Verhältnis von größter Abmessung zu kleinster Abmessung von 8:1 bis 2:1 haben.

3. Teilchenförmiges Material nach Anspruch 1 oder 2,
dadurch gekennzeichnet,
daß die Teilchen nadelförmig sind und die mittlere größte Abmessung im Bereich von 0,02 bis 0,15 Mikrometer ist.

4. Teilchenförmiges Material nach Anspruch 1,
dadurch gekennzeichnet,
daß die Titandioxidteilchen im wesentlichen kugelförmig sind und einen mittleren Durchmesser im Bereich von 0,01 bis 0,06 Mikrometer haben.

5. Teilchenförmiges Material nach Anspruch 1,
dadurch gekennzeichnet,
daß von 80 % bis 100 % der Teilchen eine Größe von 0,005 bis 0,15 Mikrometer haben.

6. Teilchenförmiges Material nach einem der vorhergehenden Ansprüche,
dadurch gekennzeichnet,
daß die Menge von Zinkoxid oder wasserhaltigem Oxid von 300 bis 1000 Gew.-% bezüglich des Gewichts von Titandioxid ist.

7. Teilchenförmiges Material nach einem der Ansprüche 1 bis 5,
dadurch gekennzeichnet,
daß die Menge von Zinkoxid oder wasserhaltigem Zinkoxid von 750 bis 2000 Gew.-% bezüglich des Gewichts von Titandioxid ist.

8. Teilchenförmiges Material nach einem der vorhergehenden Ansprüche,
dadurch gekennzeichnet,
daß, wenn das Material dispergiert ist, es einen Extinktionskoeffizienten für Licht mit einer Wellenlänge von 308 Nanometer (E(308)) von mindestens 15 Liter pro Gramm pro Zentimeter aufweist und einen Extinktionskoeffizienten für Licht mit einer Wellenlänge von 360 Nanometer (E(360)) von mindestens 10 Liter pro Gramm pro Zentimeter aufweist.

9. Teilchenförmiges Material nach einem der vorhergehenden Ansprüche,
dadurch gekennzeichnet,
daß, wenn das Material dispergiert ist, es einen Extinktionskoeffizienten für Licht mit einer Wellenlänge von 524 Nanometer (E(524)) von weniger als 5 Liter pro Gramm pro Zentimeter hat.

10. Teilchenförmiges Material nach einem der vorhergehenden Ansprüche,
dadurch gekennzeichnet,
daß das Material in der Form einer Dispersion in Öl vorliegt und die Dispersion durch Mahlen des teilchenförmigen Materials in Öl in der Gegenwart eines Dispergiermittels hergestellt ist.

11. Verfahren zum Herstellen von teilchenförmigem Material, umfassend das Bilden einer wäßrigen Dispersion von Titandioxidteilchen in der Gegenwart eines hydrolysierbaren Salzes von Zink und Bewirken der Hydrolyse des Zinksalzes, um auf den Titandioxidteilchen eine Beschichtung aus Zinkoxid oder wasserhaltigem Zinkoxid zu bilden,
dadurch gekennzeichnet,
daß das Titandioxid eine mittlere Teilchengröße im Bereich von 0,005 bis 0,15 Mikrometer hat, das Zinksalz in einer Menge verwendet wird, die ausreichend ist, um auf den Titandioxidteilchen eine Beschichtung von Zinkoxid oder wasserhaltigem Zinkoxid abzulagern, welche mindestens 100 Gew.-% bezüglich des Gewichts von Titandioxid umfaßt und worin das so gebildete beschichtete Titandioxid im wesentlichen für sichtbares Licht transparent ist und im wesentlichen Ultraviolettlicht absorbiert.

12. Verfahren nach Anspruch 11,
dadurch gekennzeichnet,
daß die wäßrige Dispersion von Titandioxidteilchen eine Konzentration von 80 bis 250 Gramm pro Liter hat.

13. Verfahren nach Anspruch 11 oder 12,
dadurch gekennzeichnet,
daß zu der Dispersion von Titandioxidteilchen ausreichend Zinkchlorid gegeben wird, um eine erwünschte Beschichtungsmenge von Zinkoxid auf den Titandioxidteilchen zu bilden und daß das Zinkoxid durch eine nachfolgende Zugabe von einem Alkali bis der pH-Wert der Dispersion zwischen 9 und 10 ist, präzipitiert wird.

14. Verfahren nach Anspruch 11 oder 12,
dadurch gekennzeichnet,
daß die Dispersion von Titandioxidteilchen bei einem pH-Wert zwischen 9 und 10 gebildet wird, die Zinkchloridlösung langsam zu dieser Dispersion gegeben wird und der pH der Dispersion bei einem Wert zwischen 9 und 10 durch die gleichzeitige Zugabe von einem Alkali gehalten wird.

15. Verfahren nach einem der Ansprüche 11 bis 14,
dadurch gekennzeichnet,
daß die Dispersion von Titandioxidteilchen einem Mahlverfahren unterworfen wird bevor mit der Beschichtung von Zinkoxid oder wasserhaltigem Oxid begonnen wird.

16. Verfahren nach Anspruch 15,
dadurch gekennzeichnet,
daß das Mahlen in der Gegenwart eines Dispergiermittels, welches ein Alkalimetallsilikat, ein Alkanolamin oder ein Polyacrylat umfaßt, durchgeführt wird.

17. Verfahren nach einem der Ansprüche 11 bis 16,
dadurch gekennzeichnet,
daß das beschichtete Titandioxid durch Wiederaufschlämmen mit Wasser bei 60°C bis 70°C gewaschen wird.

18. Verfahren nach einem der Ansprüche 11 bis 17,
dadurch gekennzeichnet,
daß das beschichtete Titandioxid weiterbehandelt wird durch Erhitzen bei einer Temperatur von 200°C bis 650°C.

## Revendications

1. Matériau particulaire comprenant du dioxyde de titane ayant un enrobage constitué d'oxyde de zinc ou d'oxyde de zinc hydraté, caractérisé en ce que les particules de dioxyde de titane ont une granulométrie moyenne comprise entre 0,005 et 0,15 µm et en ce que le revêtement d'oxyde de zinc ou d'oxyde de zinc hydraté est présent en une quantité d'au moins 100 % en poids par rapport au poids de dioxyde de titane, ledit matériau étant pratiquement transparent vis-à-vis de la lumière visible et absorbant substantiellement les rayonnements ultraviolets.

2. Matériau particulaire selon la revendication 1, caractérisé en ce que les particules de dioxyde de titane ont une forme aciculaire et ont un rapport de leur dimension maximale à leur dimension minimale compris entre 8/1 et 2/1.

3. Matériau particulaire selon la revendication 1 ou 2, caractérisé en ce que les particules ont une forme aciculaire et que leur dimension maximale moyenne est comprise entre 0,02 et 0,15 µm.

4. Matériau particulaire selon la revendication 1, caractérisé en ce que les particules de dioxyde de titane ont une forme pratiquement sphérique et ont un diamètre moyen compris entre 0,01 et 0,06 µm.

5. Matériau particulaire selon la revendication 1, caractérisé en ce que 80 à 100 % des particules ont une taille comprise entre 0,005 et 0,15 µm.

6. Matériau particulaire selon l'une quelconque des revendications précédentes, caractérisé en ce que la quantité d'oxyde de zinc ou d'oxyde de zinc hydraté est comprise entre 300 et 1000 % en poids par rapport au poids de dioxyde de titane.

7. Matériau particulaire selon l'une quelconque des revendications 1 à 5, caractérisé en ce que la quantité d'oxyde de zinc ou d'oxyde de zinc hydraté est comprise entre 750 et 2000 % en poids par rapport au poids de dioxyde de titane.

8. Matériau particulaire selon l'une quelconque des revendications précédentes, caractérisé en ce que, lorsqu'il est dispersé, le matériau a un coefficient d'extinction de la lumière à une longueur d'onde de 308 nm (E(308)) d'au moins 15 litres par gramme par centimètre et un coefficient d'extinction de la lumière à une longueur d'onde de 360 nm (E(360)) d'au moins 10 litres par gramme par centimètre.

9. Matériau particulaire selon l'une quelconque des revendications précédentes, caractérisé en ce que, lorsqu'il est dispersé, le matériau a un coefficient d'extinction de la lumière à une longueur d'onde de 524 nm (E(524)) inférieur à 5 litres par gramme par centimètre.

10. Matériau particulaire selon l'une quelconque des revendications précédentes, caractérisé en ce que le matériau se présente sous la forme d'une dispersion dans l'huile, et que la dispersion est préparée par broyage du matériau particulaire dans l'huile en présence d'un agent dispersant.

11. Procédé pour préparer un matériau particulaire, qui consiste à former une dispersion aqueuse de particules de dioxyde de titane en présence d'un sel hydrolysable de zinc, et à effectuer l'hydrolyse du sel de zinc de façon à former, sur les particules de dioxyde de titane, un enrobage d'oxyde de zinc ou d'oxyde de zinc hydraté, caractérisé en ce que le dioxyde de titane a une granulométrie moyenne comprise entre 0,005 et 0,15 µm, en ce que le sel de zinc est utilisé en une quantité suffisante pour déposer sur les particules de dioxyde de titane un enrobage d'oxyde de zinc ou d'oxyde de zinc hydraté représentant au moins 100 % en poids de la quantité de dioxyde de titane, et en ce que le dioxyde de titane enrobé ainsi formé est pratiquement transparent vis-à-vis de la lumière visible et absorbe substantiellement les rayonnements ultraviolets.

12. Procédé selon la revendication 11, caractérisé en ce que la dispersion aqueuse de particules de dioxyde de titane a une concentration de 80 à 250 grammes par litre.

13. Procédé selon la revendication 11 ou 12, caractérisé en ce qu'on ajoute à la dispersion de particules de dioxyde de titane suffisamment de chlorure de zinc pour former un enrobage d'oxyde de zinc sur les particules de dioxyde de titane en une quantité souhaitée, et en ce que l'oxyde de zinc précipite par addition ultérieure d'un alcali jusqu'à ce que le pH de la dispersion soit compris entre 9 et 10.

14. Procédé selon la revendication 11 ou 12, caractérisé en ce que la dispersion de particules de dioxyde de titane est formée à un pH compris entre 9 et 10, en ce qu'on ajoute lentement la solution de chlorure de zinc à cette dispersion et en ce que le pH de la dispersion est maintenu entre 9 et 10 par addition simultanée d'un alcali.

15. Procédé selon l'une quelconque des revendications 11 à 14, caractérisé en ce qu'on soumet la dispersion de particules de dioxyde de titane à un processus de broyage avant que commence la formation de l'enrobage d'oxyde de zinc ou d'oxyde de zinc hydraté.

16. Procédé selon la revendication 15, caractérisé en ce qu'on effectue le broyage en présence d'un agent dispersant comprenant un silicate de métal alcalin, une alcanolamine ou un polyacrylate.

17. Procédé selon l'une quelconque des revendications 11 à 16, caractérisé en ce qu'on lave le dioxyde de titane enrobé en le remettant en suspension dans de l'eau à 60-70°C.

18. Procédé selon l'une quelconque des revendications 11 à 17, caractérisé en ce qu'on traite ensuite le dioxyde de titane enrobé en le chauffant à une température de 200 à 650°C.
